# EUROPEAN PATENT APPLICATION

(11) **EP 2 594 546 A1**
(43) Date of publication of application: **22.05.2013**
(21) Application number: 11189629.6
(22) Date of filing: 17.11.2011
(51) Int. Cl.: C07C 1/24, C07C 7/148, C07C 11/04, C07C 17/02

(54) **Process for the manufacture of ethylene by dehydration of ethanol**

(71) Applicant: SOLVAY SA, 1120 Bruxelles (BE)
(72) Inventor: The designation of the inventor has not yet been filed
(74) Representative: Vande Gucht, Anne

(57) **Abstract**

To this effect, the present invention relates to a process for the manufacture of ethylene by dehydration of ethanol and preferably, of a biomass-derived ethanol also termed `bioethanol' or 'hydrous ethanol', said process comprising the steps of:
a) subjecting a feedstock comprising ethanol to dehydration to produce an olefins stream comprising ethylene and water;
b) drying at least part of the olefins stream;
said process being characterized by the fact of adding a reducing agent to the at least part of the olefins stream prior to drying it.

## Description

The present invention relates to a process for the manufacture of ethylene by dehydration of ethanol (preferably biomass-derived ethanol feedstock), in particular in view of manufacturing polyethylene or 1,2-dichloroethane (DCE) and optionally therefrom, vinyl chloride and subsequently, polyvinyl chloride.

One of the problems faced by the manufacture of polymers from ethylene is that the starting raw materials are from fossil fuels (natural gas, crude oil) which are non-renewable feedstocks.

Applicants however have realized that the production of ethylene and ethylene derivatives compounds would benefit by the replacement of at least a part of the carbonaceous raw materials of fossil origin by renewable resources, such as carbonaceous matter derived from biomass. Of particular interest is the ethanol feedstock which is produced from renewable resources. Such biomass-derived ethanol, also referred to as 'bioethanol' or 'hydrous fuel alcohol' can be prepared in large quantities from biomass via fermentation. The different feedstocks for producing ethanol may be sucrose-containing feedstocks (e.g., sugarcane), starchy materials (e.g., corn, starch, wheat, cassava,... ), lignocellulosic biomass (e.g., switchgrass) and/or agricultural waste. The purification or isolation of bioethanol is frequently carried out by complicated, multistage distillation.

Even after the known purification processes, the advantage of bioethanol is frequently decreased by small amounts of impurities it contains. Bioethanol impurities may include oxygen-containing organics, for example other alcohols such as isopropanol, n-propanol, and isobutanol, and/or aldehydes such as acetaldehyde. Bioethanol impurities may further include sulfur-containing impurities, such as inorganic sulfur compounds (sulfite, sulfate) C₂-10-dialkyl sulfides, C₂-10-dialkyl disulfides, C₂-10-dialkyl sulfoxides, C₂-10-alkyl mercaptans, 3-methylthio-1-propanol, and/or S-containing amino acids.

Some of these impurities may interfere with the downstream processes which use bioethanol as feedstock and which generate chemical products, especially when some of the downstream steps are catalytic conversions.

If efforts are not made to remove at least some of these impurities, the yield of desired intermediate and final products (ethylene, at least one ethylene derivative compound or any compound derived therefrom) and efficacy of the overall process may be diminished.

To date, ethylene which is typically used for the manufacture of polymers comprising ethylene and of 1, 2-dichloroethane (DCE) is more than 99.8 % pure (i.e., contains impurities of 0.2 % or less). Ethylene of very high purity is in high demand for production various polyethylene polymers, and may be sometimes termed 'polyethylene-grade' ethylene.

Processes for dehydration of ethanol to ethylene are well known. These processes typically require temperatures in excess of 300°C where both the olefin and alcohol are in the gas phase and achieve near complete or essentially complete conversion of alcohol to olefin. The thermodynamics favor such high alcohol to olefin conversions only at low pressure, so the process is conventionally operated at or just above atmospheric pressure (e.g., 1 to 2 atm). However, renewable ethanol still contains water, and the presence of water is thermodynamically detrimental to achieving a high conversion in gas-phase ethanol-to-ethylene dehydration.

In a typical gas-phase dehydration process, ethanol is first vaporized and fed to a dehydration reactor (e.g., containing two vessels generally operated in alternating mode to allow regeneration of catalyst) at about 1 atm. Since the dehydration reaction is endothermic, there is an input of heat provided to the reactor. A hot ethylene stream exiting the dehydration reactor is quenched with water and is generally scrubbed with caustic. The ethylene produced must generally meet critical purity specifications. Purification is typically done via cryogenic distillation at elevated pressure, so when ethylene is produced by the gas phase dehydration of ethanol, it must be compressed before purification. For example the quenched and scrubbed ethylene stream is compressed to a pressure from about 0.1 MPa to about 3 MPa, caustic washed, and dried. The dried ethylene stream -may be fed to a demethanizer to remove C 1 hydrocarbons, and finally to a 'C2' splitter where an ethylene product is produced as an overhead stream, and wherein C3+ are purged. The ethylene produced after purification may be again compressed to the operating pressure of the eventual downstream process.

As a result of the dehydration of ethanol, it should be noted that by-products such as alkanes (e.g., methane, ethane), aldehydes (e.g, acetaldehyde), ketones, ethers (e.g., diethylether), and oligomers may be formed. Some byproducts can be formed by coupling of alkyl fragments e.g., acid catalysed olefin oligomerisation, such as: 2 propylene → hexene. Some by-products may be formed by alcohol dehydrogenation, e.g., ethanol → acetaldehyde + H₂. The acetaldehyde level in the final ethylene is also influenced by the impurities of the ethanol feedstock.

In the presence of caustic, the acetaldehyde present in the ethylene stream will undergo an aldol condensation and polymerization which will plug the caustic column which generally follows (or is integrated with) the water-quench column. This problem is known for many years (see for instance KOCHAR, N., MERIMS, R. & PADIA, A. (1981) Ethylene from ethanol. CEP (June)). However, to date, the solution thereto has been to purify (for instance by distillation) the feedstock ethanol which is costly and time consuming, especially if pushed far enough to get only few traces of acetaldehyde.

Another problem encountered when the ethylene stream produced contains acetaldehyde is the fouling of the aforementioned C2 splitter.

The present invention is based on the idea of getting rid of some impurities (especially aldehydes like acetaldehyde) during the ethylene manufacturing process itself.

To this effect, the present invention relates to a process for the manufacture of ethylene by dehydration of ethanol and preferably, of a biomass-derived ethanol also termed 'bioethanol' or 'hydrous ethanol', said process comprising the steps of:
a) subjecting a feedstock comprising ethanol to dehydration to produce an olefins stream comprising ethylene and water;
b) drying at least part of the olefins stream;
   said process being characterized by the fact of adding a reducing agent to the at least part of the olefins stream prior to drying it.

The process according to the invention preferably uses an impure ethanol feedstock. The impure ethanol feedstock may be anhydrous or hydrous, preferably hydrous. The ethanol feedstock preferably comprises biomass-derived ethanol. The biomass-derived ethanol may be anhydrous or hydrous, preferably hydrous bioethanol. The ethanol feedstock most preferably consists essentially of hydrous bioethanol.

The hydrous bioethanol may have an ethanol content of at least 90%, preferably between 92 and 95%, more preferably between 92.5 and 94%. The hydrous bioethanol may have a water content of from 3 to 8% water, preferably between 5 and 7%. The density of this hydrous bioethanol may be between 807 and 811 kg/m³.

Impurities in the hydrous bioethanol may include oxygen-containing compounds, also termed 'oxygenates':
- aldehydes such as acetaldehyde, crotonaldehyde;
- other alcohols, such as methanol, isopropanol, n-propanol, n-butanol, isobutanol, isoamylic alcohol;
- alkanes, such as n-hexane, cyclohexane;
- ketones, such as acetone; and/or
- esters, such as ethyl acetate.

The impurities in such hydrous bioethanol may include sulfur-containing compounds:
- inorganic sulfur, such as sulfate which can be added for pH control during fermentation or sulfite which can be utilized for treatment of biomass juice for the production of refined sugar;
- symmetrical or unsymmetrical C₂-10-dialkyl sulfides, particularly C₂-6-dialkyl sulfides (such as diethyl sulfide, di-n-propyl sulfide, diisopropyl sulfide), very particularly dimethyl sulfide;
- symmetrical or unsymmetrical C₂-10-dialkyl disulfides, particularly C₂-6-dialkyl disulfides, such as dimethyl disulfide;
- symmetrical or unsymmetrical C₂-10-dialkyl sulfoxides, such as dimethyl sulfoxide, diethyl sulfoxide, dipropyl sulfoxide;
- C₁-10-alkyl mercaptans, such as methyl mercaptan, ethyl mercaptan, n-propyl mercaptan;
- 3-methylthio-1-propanol; and/or
- S-containing amino acids, such as methionine and S-methylmethionine.

Other impurities in such hydrous bioethanol may include compounds containing other heteroatoms chosen from Fe, Na, or Cl.

According to the invention, dehydration may employ a vapor-phase or liquid-phase reaction which takes place in a dehydration reactor.

When the dehydration uses a vapor-phase reaction, the ethanol feedstock may be vaporized (for example by the use of steam) and fed to a dehydration reactor.

The dehydration reactor may be operated under isothermal or adiabatic conditions, preferably adiabatic conditions.

The dehydration reactor or reaction zone may contain a fluidized bed or a fixed bed.

Since the dehydration reaction is endothermic, there needs to be an input of heat provided to the reactor. For a single bed adiabatic reactor the overall endothermic reaction if allowed to go to thermodynamic equilibrium could result in a theoretical temperature drop of 180 °C. Heat management can be addressed by reactor design. Suitable reactor designs include those capable of handling heat fluxes such as fixed bed, fluidized bed, multi-tubular and multiple fixed bed reactors with inter-stage heating zones. Optionally the heat management can be improved by injecting preheated fresh alcohol feed at several points in the reactor bed. The ethanol feedstock may be preheated for example in a furnace to a temperature above the reaction temperature (e.g., at about 400°C), in order to provide an additional source of heat. A portion of an ethanol recycle stream also can be added at several points along the reactor with additional heating ; alternatively the main portion of such recycle stream may be added to the front end of the reactor. In embodiments when a plurality of dehydration reaction zones operates in series, the gas effluent exiting a reaction zone is heated prior to entering the subsequent reaction zone; such intermediate heating can be done by passing the gas effluent in the same furnace through which the fresh ethanol feedstock is preheated (albeit in separate flow paths).

The dehydration reactor may comprise more than one dehydration reaction zone. Two or more dehydration reaction zones may be operated in series with inter-stage heating zones sandwiched between two consecutive dehydration reaction zones to allow intermediary heat input. Alternatively, the two or more dehydration reaction zones may be operated in parallel so as to allow regeneration of dehydration catalyst contained herein. The different dehydration reaction zones may be housed in separate vessels or in a single vessel.

An example of an isothermal reactor includes isothermal tubular reaction zones inside which the dehydration reaction takes place and outside which a heating medium circulates to provide heat of dehydration reaction.

An example of an adiabatic reactor includes a reactor inside which two or more dehydration reaction zones are located. The adiabatic dehydration reactor preferably comprises a plurality of dehydration reaction zones operated in series (for several consecutive dehydration stages) in form of fixed beds and one or more inter-stages zones sandwiched between two consecutives fixed beds where additional heat may be provided. As an effluent gas exits a first dehydration reaction zone to be directed to a second dehydration reaction zone, it may be heated *in situ* in the inter-stage zone positioned immediately downstream of the first dehydration reaction zone and immediately upstream of a second dehydration reaction zone or may be heated *ex situ* by passing through an external heating zone (e.g., furnace) which may be different or preferably the same as the one through which the ethanol feedstock is preheated, and such heated effluent gas serves as feedstock to the second dehydration reaction zone.

Ethylene production from ethanol dehydration is a widely known process. In this process, ethanol is converted into ethylene by means of a catalytic reaction at a temperature of at least 180°C, preferably of at least 300°C.

In preferred embodiments, step (a) comprises contacting the feedstock comprising the biomass-derived ethanol with a dehydration catalyst at a temperature of at least 300°C, more preferably from 310 to 450°C.

In other embodiments, step (a) comprises contacting the feedstock comprising the biomass-derived ethanol with a dehydration catalyst at a temperature of at least 180°C but less than 300°C.

In preferred embodiments, step (a) comprises contacting the feedstock ethanol with a dehydration catalyst at a pressure from 0.5 atm to about 25 atm absolute (from 50 kPa to 2.5 MPa), preferably from 1 to 5 atm (from 101 to 505 kPa), most preferably from 1.1 to 3 atm (from 110 to 303 kPa). Alternate embodiments may comprise a dehydration operating pressure of from 2 to 25 atm (from 202 to 2525 kPa), or from 2 to 20 atm (from 202 to 2020 kPa), or even from 2 to 15 atm (from 202 kPa to 1515 kPa).

The dehydration step (a) is carried out with a dehydration catalyst comprising an acidic component. A large variety of acid catalysts can be used for the dehydration step (a), but the most commonly used solid types include pure or doped high specific surface area gamma-alumina (γ -Al₂O₃), titania/ y - alumina, magnesium oxide, H-mordenites, silica-aluminate such zeolites (e.g., H-ZSM 5), silica/titania, silicoaluminophosphates ('SAPO' catalysts), and silica-alumina. Other catalysts may be used for liquid-phase dehydration. Preferred examples are solid acid catalysts including isomorphously substituted aluminum phosphate (AIPO) catalysts such as disclosed in WO 2010/085708 (incorporated herein by reference). Unsubstituted AIPO's (see US 3,915,893 incorporated herein by reference) and other strong solid acids such as SAPO's, silicalites, and zeolites (e.g., Zeolite ZSM-5) can be employed. Other nonvolatile acids, such as sulfuric acid, p-toluenesulfonic acid, methanesulfonic acid, heteropoly acids or phosphoric acid may also be used in the dehydration step.

It should be noted that during the dehydration of ethanol, by-products are generally formed. These can be formed by coupling of alkyl fragments, e.g., acid catalyzed olefin oligomerization, such as 2 propylene → hexene; or by alcohol dehydrogenation, such as ethanol → acetaldehyde + H₂₁ or transfer hydrogenation reaction, such as ethylene + H₂ → ethane. The formation of same carbon number alkanes is known to add significantly cost in separation in order to produce polyethylene-grade ethylene. However, alkanes presence in the ethylene stream does not necessarily require their removal, as some processes which produce ethylene derivatives may not be negatively impacted by the presence of alkanes in ethylene. For example, it has been found that the presence of alkanes (e.g., ethane) in the ethylene stream does not poison or impede an oxychlorination reaction. Thus to make DCE as one ethylene derivative via oxychlorination, there is no need to separate ethane from ethylene by way of very expensive fractionation. The same is true for oxygenates, as it has been found that the oxychlorination reaction is not impacted by the presence of oxygenates in the ethylene product formed via dehydration.

However oxygenates (e.g., acetaldehyde and alcohols) in the ethylene feedstream can negatively impact a chlorination reaction, and as such at least a portion of these oxygenates need to be removed from the portion of the ethylene stream which is fed to the chlorination reactor. These oxygenates must also be at a low level in polymer grade ethylene for polyethylene plants.

It results from the above that the olefins stream generated in step a) of the process of the invention contains ethylene, water, unconverted ethanol and the above mentioned impurities.

This stream is preferably quenched before being dried in step (b) of the invention, in an appropriate quench device, generally a column.

The quench device is generally fed from vapor effluent of a waste heat boiler which receives the hot dehydration reactor effluent. As used herein, "quench device" is a device for removing some components of the reactor effluent by establishing a sufficient quantity of liquid quench medium in contact with a gaseous dehydration reactor which condenses at least a portion of some components from the reactor. A "quench medium" is defined as a liquid that contacts the dehydration reactor effluent in a quench device. Examples of a quench device in an oxygenate-to-olefin product stream can be found in U.S. Pat. No. 6,121,504 (direct product quench) and U.S. Pat. No. 7,005,555 (removal of oxygenate(s) including carbon dioxide in the bottoms of the quench stream and recycle of the oxygenate(s) back to the reactor), each fully incorporated herein by reference.

The quench device in one embodiment may have a single stage or multiple stages. The multiple-stages quench device of a particular embodiment preferably has two to four stages, more preferably two to three stages. The quench device is optionally in single or multiple housings or towers.

The quench device typically includes internal elements in a contact zone to facilitate intimate contacting of the quench medium with the reactor effluent or portions thereof. Contact zone is defined as the zone of the quench device where the dehydration reactor effluent comes in contact with the quench medium. Internal elements include liquid distributors and contacting devices such as baffles, trays, random packing, or structured packing.

As used herein, the term, "quench bottoms stream" refers to the portion of the reactor and quench medium that is liquid under quench conditions and includes all streams that contain the condensed portion of the olefins stream and fractions of the condensed olefins stream. The term "quenched olefins stream" refers to the portion of the reactor olefins stream that is gaseous after at least one step of quenching.

The quenched olefin stream is predominantly gaseous and, in preferred sub-embodiments, leaves the quench device. The quench bottoms stream may contain a majority of catalyst fines which may be present in the dehydration reactor. The quench bottoms stream is withdrawn optionally for additional processing. In one embodiment, a quench medium is supplied to the quench device. According to one embodiment, the quench medium is an aqueous quench medium. In another embodiment, the quench medium is an aqueous quench medium that is pH adjusted by adding an alkaline, preferably caustic i.e. a strong alkaline like NaOH or KOH, the first one (NaOH) being more commonly used. In still another embodiment, the pH is adjusted by adding ammonium or amines. Finally, use can be made of at least two different quench media in at least 2 different housings or towers, the first one being water (a neutral aqueous medium) and the second one being an aqueous quench medium that is pH adjusted by adding an alkaline, preferably caustic.

According to a preferred embodiment, when the pH of the quench medium is adjusted, it is greater than 7 as it enters the quench device. Preferably, the pH of the quench medium as it enters the quench device ranges from about 7.1 to about 11.5, more preferably ranges from about 7.5 to about 11, even more preferably ranges from about 8 to about 10 at the time the quench medium enters the quench device. Most preferably, the pH of the quench medium as it enters the quench device is about 9.

The quench device bottoms temperature is from about 180 °F. (82°C.) to about 300 °F. (149 °C.); preferably from about 180 °F. (82°C.) to about 250 °F. (121 °C.); more preferably about 200 °F. (93 °C.). The temperature of the quench medium is from about 60 °F. (15 °C.) to about 200 F. (93 °C.); preferably from about 80 F. (27 °C.) to about 140 F. (60 °C.); more preferably about 110 °F. (43 °C.). The quench device may be operated at a pressure that is from about 2 bars (200 kPa) to about 4.5 bars (450 kPa); preferably from about 2 bars (200 kPa) to about 3.77 bars (377 kPa); and more preferably at about 2.4 bars (240 kPa).

The optional step of contacting the olefins stream (exiting the dehydration reactor) with a quench medium removes water from the olefins stream. In one embodiment, the step of contacting the olefins stream with a quench medium removes 95 wt. % or more of the water, preferably 98 wt. % or more, more preferably 99 wt. % or more from the olefins stream based upon the total amount of water in the olefins stream before the olefins stream is quenched. However, in this embodiment of the invention, the quench step does not remove substantially all the water (since a drying (step (b)) is still required). The amount of water remaining after the quench can be up to 0.1 w% (or 1000 wppm) and even up to 0.5 w% (or 5000 wppm).

In one embodiment, a majority of catalyst fines present in the olefins stream may be removed in the quench bottoms stream of the quench device. The term "majority" means more than 50%. According to one embodiment, the weight of the catalyst fines in the quench bottoms stream is about 5 wt. % or less, preferably about 2 wt. % or less, more preferably about 0.1 wt. % or less based on the total weight of the quench bottoms stream. According to one embodiment, the weight of catalyst fines in the quench bottoms stream is about 10 wppm or greater based upon the total weight of the quench bottoms stream.

There is another embodiment according to any process disclosed herein wherein the step of contacting the olefins stream with a quench medium removes 5 wt. % or more, preferably 50 wt. % or more, more preferably 75 wt. % or more, most preferably about 80 wt. % of the carbon dioxide from the olefins stream based upon the total amount of carbon dioxide in the olefins stream before the olefins stream is quenched.

Generally, contacting the olefins stream with a quench medium removes from about 25 wt. % to about 95 wt. %, preferably from about 40 wt. % to about 90 wt. %, more preferably from about 50 wt. % to about 85 wt. %, of the aldehydes and/or ketones from the olefins stream based upon the total amount of aldehydes and/or ketones in the olefins stream before the olefins stream is quenched.

Oxygenates, including but not limited to unreacted ethanol feed, particularly bioethanol feed, is found in the olefins stream exiting the dehydration reactor typically when oxygenate conversion to olefin in the dehydration reactor is less than 100%. In one embodiment, the olefins stream further comprises one or more alcohols other than ethanol. Oxygenates, including but not limited to unreacted bioethanol, are at least partially removed from the olefins stream in the quench column. The step of contacting the olefins stream with a quench medium removes 90 wt. % or more, preferably 95 wt. % or more, more preferably 98 wt. % or more, most preferably 99 wt. % or more of alcohols from the olefins stream based upon the total amount of alcohol in the olefins stream before the olefins stream is quenched.

At least a portion of the quenched olefins stream is gaseous after at least one step of quenching, typically comprises light olefin(s) including ethylene, propylene and butylene, dimethyl ether, methane, carbon monoxide, ethane, propane, and any water and unreacted oxygenate(s) such as alcohol (including methanol) that are not condensed during the operation of the quench device. According to some embodiments of the present invention, not all carbon dioxide is removed from the reactor by the step contacting the olefins stream with a quench medium.

According to the invention, the olefins stream, eventually quenched, is dried, eventually after having been compressed first. If the dehydration and/or quench conditions are such that the olefins stream exiting the top of the reactor or the quench column, the case being, is superatmospheric then it must not necessarily be subjected to a compression step

If it is compressed, the resulting pressure of the compressed olefins stream may be at least 1 bars (100 kPa), or at least 1.1 bars (110 kPa), or at least 2 bars (200 kPa), or at least 3 bars (300 kPa), or at least 4 bars (400 kPa). The resulting pressure of the compressed olefins stream may be 25 bars or less (2500 kPa or less), or 20 bars or less (2000 kPa or less), or at most 15 bars or less (1500 kPa or less).

The optional compression may be performed in a single stage of compression or may be performed in several stages, either in a multi-stage gas compressor or in several compressors. The compression ratio at each compression stage is such that the temperature at the exit of the compression stage is advantageously of at most 150 °C, preferably of at most 120 °C and more preferably of at most 100 °C.

The gas which exits the compression stage may be advantageously cooled down afterwards by indirect cooling with a cooling media. The cooling media is advantageously chosen among cooling tower water, cold water, atmospheric air and colder gas issued from the process. The cooling media may be preferably chosen among cooling tower water and atmospheric air. The cooling fluid is more preferably cooling tower water.

The compressed olefins stream may be advantageously cooled down under 50°C, preferably under 48°C and more preferably under 45°C but advantageously not under 0°C, preferably not under 5°C and more preferably not under 10°C. At the end of the cool down, some condensates (e.g., water) may be produced. If some condensate is produced, it is preferably separated.

The drying step (b) of the process of the invention may include passing at least part of the olefins stream through a drying unit in order to obtain a dry ethylene-containing stream. By the term "dry" is meant that the ethylene-containing stream contains less than about 100 wppm water, more preferably less than about 10 wppm, and most preferably less than 1 wppm based upon the weight of the olefin product stream.

Any suitable means may be used for drying. The drying step (b) may include (eventually further) compression, fractionation, solid or liquid adsorption, or combinations of two or more of these techniques. A solid adsorption for example may include a molecular sieve adsorption.

When the drying step (b) includes a fractionation, such fractionation may be performed by a distillation column in which the heavies (e.g., water) are collected at the bottoms and ethylene is mainly separated into a light fraction.

When the drying step (b) includes an adsorption, such adsorption may be performed by solid or liquid adsorption to remove water from the ethylene-containing stream. In the solid drying system, the olefins stream is contacted with a solid adsorbent to further remove water to very low levels. Typically, the adsorption step is carried out in one or more fixed beds containing a suitable solid adsorbent.

Adsorption is useful for removing water and also oxygenates to very low concentrations, and for removing oxygenates that may not normally be removed by using other treatment systems. Preferably, an adsorbent system may have multiple adsorbent beds. Multiple beds allow for continuous separation without the need for shutting down the process to regenerate the solid adsorbent. By way of example and not by limitation, a three bed system typically has one bed that is on-line, one bed regenerated off-line, and a third bed on stand-by.

The specific adsorbent solid or solids used in the adsorbent beds depends on the types of contaminants being removed. Non-limiting examples of solid adsorbents for removing water and various polar organic compounds, such as oxygenates and absorbent liquids, include calcium chloride, aluminas, silica, 3A molecular sieves, 4A molecular sieves, and alumino-silicates. Beds containing mixtures of these sieves or multiple beds having different adsorbent solids are used to remove water, as well as a variety of oxygenates in one embodiment.

In an embodiment of the present invention, one or more adsorption beds are arranged in series or parallel. In one example of a series arrangement, a first bed is used to remove the smallest and most polar molecules, which are the easiest to remove. Subsequent beds for removing larger less polar oxygenated species are next in series. As a specific example of one type of arrangement, water is first selectively removed using a 3A molecular sieve. This bed is then followed by one or more beds containing one or more less selective adsorbents such as a larger pore molecular sieve, e.g., 13x ; and/or a high surface area active alumina.

In another embodiment, the first bed is a 3.6 A molecular sieve capable of selectively separating both water and methanol from the hydrocarbons in the olefin stream. This first bed can then be followed by one or more 13x; or active alumina beds as described above.

The adsorbent beds can be operated at ambient temperature or at elevated temperature as required, and with either upward or downward flow.

Regeneration of the adsorbent materials can be carried out by conventional methods including treatment with a stream of a dry inert gas such as nitrogen at elevated temperature.

In the liquid adsorption drying system, a water absorbent is used to remove water from the ethylene-containing stream. The water absorbent can be any liquid effective in separating water from an olefin stream.

When the drying step (b) includes a downstream compression and an adsorption, the compression is preferably carried out prior to the adsorption.

When the drying step (b) includes an adsorption and a fractionation, the adsorption is preferably carried out prior to the fractionation.

When the drying step (b) includes a downstream compression, an adsorption, and a fractionation, the downstream compression is preferably carried out first in the series, the adsorption is carried out second and the fractionation is carried out third.

According to the invention, prior to the drying step (b), a reducing agent is added to the olefins stream. This is because most of the available reducing agents require being in an aqueous phase to be active.

A reducing agent (also called a reductant or reducer) is an element or compound that in a reduction-oxidation (redox) reaction, donates an electron to another species. In the frame of the invention, preferred reducing agents are those able to reduce aldehydes and ketones, especially aldehydes like acetaldehyde.

Since it is preferred to operate the quench (the case being) at least partially in alkaline conditions (see above), preferred reducing agents according to the invention are those active in alkaline media. Examples of such reducing agents are metal hydrides, like lithium aluminum hydride and sodium borohydride, and reducing sulfur compounds like sodium sulfite, ammonium sulfite, sodium bisulfite, potassium bisulfite, ammonium bisulfite, sodium metabisulfite, and potassium metabisulfite sodium bisulfite. Sodium borohydride and sodium bisulfite are preferred, especially the former one.

The contact with a metal hydride as described above typically results in removing at least some aldehydes such as acetaldehyde present in this ethylene-containing stream by a reduction reaction which can be carried out in an alkaline medium.

In particular, sodium borohydride (NaBH₄) is generally used in basic aqueous solution such as a sodium hydroxide solution whose concentration may vary between 0.1 M/1 and 10 M/1 and preferably between 1 and 5 M/1. The concentration of NaBH₄ in this alkaline solution is generally between 0.01 M/1 and the solubility limit of NaBH₄ in this alkaline solution; however, the concentration of NaBH₄ is preferably between 0.1 and 1 M/1. For this reduction step, the temperature conditions may be between 10 °C and 70 °C, preferably between 15 °C and 35 °C. With regard to the pressure conditions, these are not critical and are usually between 1 and 10 bars (between 100 and 1000 kPa) in such treatment, but may be higher.

The contact sodium bisulfite (NaHSO₃ typically results in removing at least some aldehydes such as acetaldehyde present in this ethylene-containing stream. When the treatment is carried out with sodium bisulfite, the operation is advantageously carried out at a pH which is controlled and, in all cases, lying between the values of 5 and 9 in order to avoid the formation of excessively large quantities of SO₂ in the reaction mixture. The pH of the mixture will be preferably maintained between the values included between 6.5 and 8.5, more preferably between 7 and 8, most preferably at about 7.5. The pH control may be ensured by any means allowing this effect to be achieved. A means which has given good results consists in adding sodium sulfite in sufficient quantities to attain the desired pH.

The NaHSO₃ concentration is generally between 0.01 M/1 and the solubility limit of this compound in water in the operating conditions of temperature and pressure However, the concentration of sodium bisulfite is preferably between 0.05 and 1 M/1. The alkali metal bisulfite treatment can be carried out in temperature conditions which are usually between -15 °C and 40 °C, preferably between -10 °C and 35 °C. With regard to the pressure conditions, these are not critical and are usually between 1 and 10 bars (between 100 and 1000 kPa) in such treatment, although higher pressures may be used. This treatment usually performed at a pressure which is lower or about equal that of the pressure conditions for the second reactor.

The use of sodium borohydride (NaBH₄) is preferred because this reducing agent also allows to remove sulfur and other impurities which sodium bisulfite (NaHSO₃ does not allow to do.

According to a preferred sub-embodiment of the invention, the reducing agent is at least partly added directly in the quench device, and preferably, in the one working with an alkaline quench medium, the case being. This embodiment allows performing the reduction reaction together with (and in the same device as) another preferred step of the process according to the invention.

In an even more preferred sub-embodiment, the reducing agent is also introduced in a second alkaline washing section with the advantage of limiting the amount of valuable reducing agent disposed with the purge flow of the first stage. A first stage could contain just enough reducing agent to prevent aldehyde polymerization with the second one used as a finisher to increases ethylene purity.

In a preferred embodiment, the olefins stream dried in step (b) is further purified by substantially removing all the C3+ therefrom. It is namely so that depending on its use, the ethylene produced by the process described can be further purified as described in the preamble of the application, for instance by being sent to a 'C2' splitter (e.g. a cryogenic distillation unit) where a pure ethylene product is produced as an overhead stream, and wherein C3+ are purged.

In one embodiment, the present invention also concerns the use of ethylene produced by the process described above for making polyethylene by polymerization of said ethylene.

In another embodiment, the present invention also concerns the use of ethylene produced by the process described above for making DCE by direct chlorination and/or oxychlorination.

In still a further embodiment, the present invention concerns a process for the manufacture of DCE comprising the steps of:
a) subjecting a feedstock comprising ethanol to dehydration to produce an olefins stream comprising ethylene and water;
b) adding a reducing agent to at least part of this olefins stream to obtain a reduced olefins stream;
c) drying said reduced olefins stream to obtain a dry reduced olefin stream;
d) feeding at least part of said dry reduced olefins stream to a direct chlorination unit for making DCE.

In this embodiment, that part of the olefins stream which is not reduced and dried, the case being, is preferably fed to an oxychlorination unit for making DCE in an integrated unit making DCE both by direct chlorination and by oxychlorination. Besides, part of the dry reduced olefins stream can also be fed to the oxychlorination unit.

Advantageously, the DCE manufactured with the process according to the embodiments described above is used to manufacture vinyl chloride monomer (VCM) which in turn, is advantageously used to manufacture polyvinyl chloride or PVC.

The present invention is illustrated in a non limitative way by Figures 1 to 3 attached, which show 3 preferred embodiments of the invention, namely:
- Fig. 1: A flow chart of a process according to an embodiment of the invention using only one quench device
- Fig. 2: A flow chart of a process according to an embodiment of the invention using 2 quench devices
- Fig. 3: A flow chart of a process similar to the one of Fig. 2 but wherein the stream coming from the water quench is split in two parts: one going to an oxychlorination unit and the other going to a direct chlorination unit after pretreatment.

In these figures, identical numbers designate identical or similar process steps, namely:
1: heating
2: dehydration
3: heat recovery
4: quench
5: compression
6: cooling
7: drying
8: purification

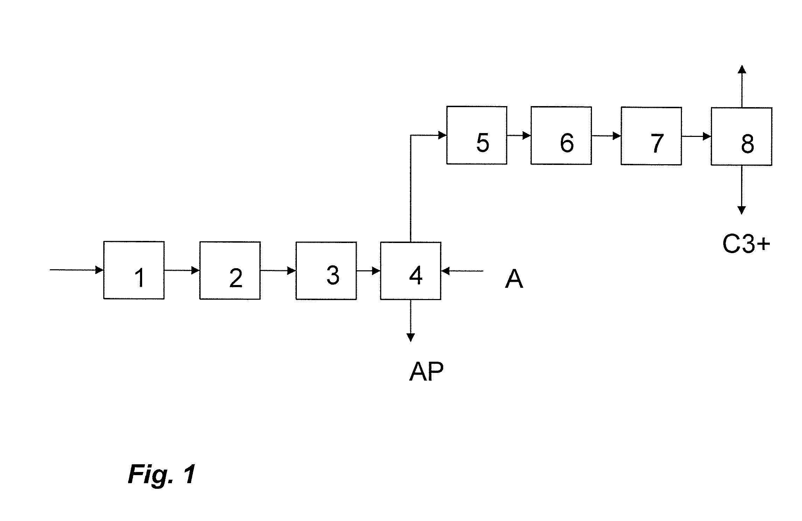

In Figure 1, a liquid ethanol feedstock is heated in a furnace in step 1, generating an ethanol vapor which is dehydrated in a reactor (step 2), generating a vapor stream comprising ethylene, water, unconverted ethanol and impurities, among which oxygenates like acetaldehyde.

This vapor stream is first sent to a waste heat boiler in order to recover heat and generate vapor in step 3. It is then sent to a quench tower where it is contacted with an alkaline (A) quench medium (step 4), from which an alkaline liquid (AP) is purged at the bottom. The quenched vapor leaving at the top of this column is first compressed in step 5 then cooled down in step 6 and finally, dried in step 7 before being sent to the final purification unit, where the C3+ are removed in step 8.

According to the invention, a reducing agent is added after step 2 (dehydration) but before step 7 (drying). Preferably, said reducing agent is NaBH4 or NaHSO₃, preferably NaBH4. Also preferably, the reducing agent is added in step 4 (alkaline quench step).

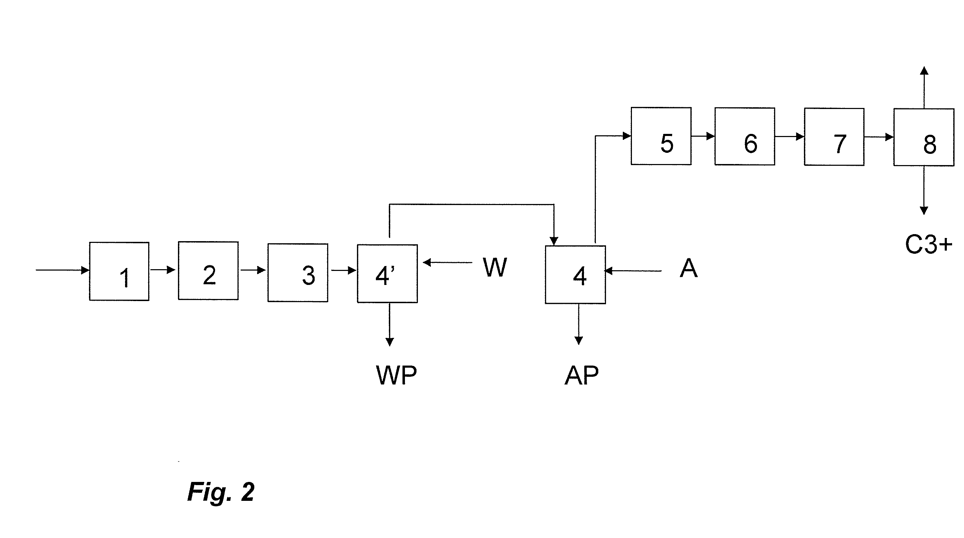

The process of Figure 2 is similar to the one of Figure 1 in all steps, except for the quench which happens in 2 steps: a water quench step (4') (using water (W) as quench medium and generating a water purge (WP)) and an alkaline quench step (4). In this embodiment, the reducing agent (which is preferably as described above) is also preferably added in step 4 (during the alkaline quench).

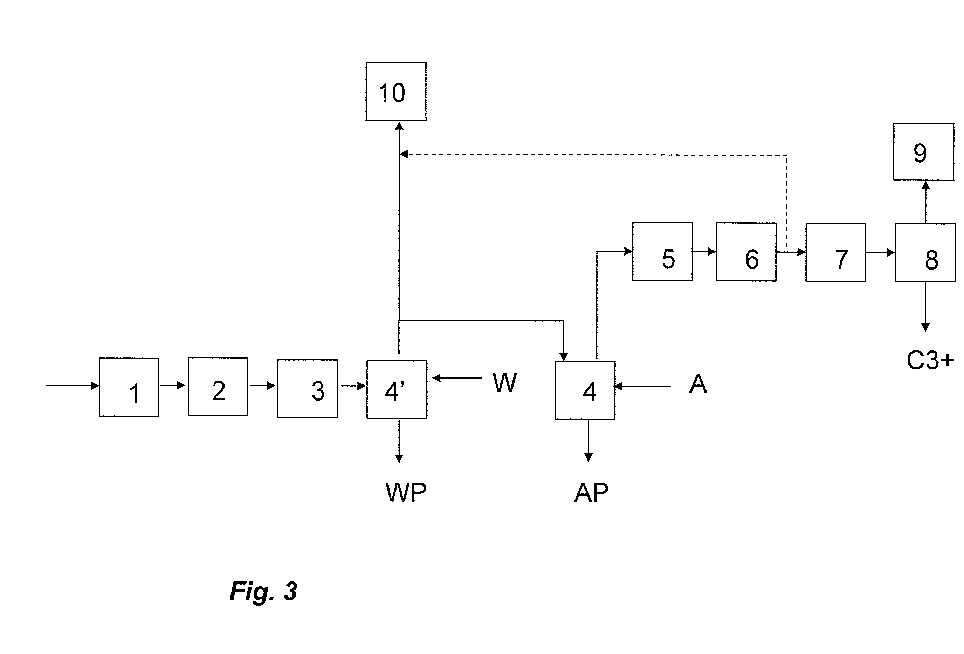

The process of Figure 3 comprises steps 1 to 8 similar to those of Figure 2, except that the vapor stream leaving the water quench step 4' is split in 2 parts, one of which being directly (without being submitted to steps 5 to 8) sent to an oxychlorination unit in order to produce DCE in step 10; and the other one being first submitted to steps 5 to 8 and then, sent to a direct chlorination unit in order to produce DCE in step 9. Optionally (as shown by the dotted lines), part of the stream leaving the cooling step 6 can be routed to the oxychlorination step 10.

## Claims

1. Process for the manufacture of ethylene by dehydration of ethanol, said process comprising the steps of:
a) subjecting a feedstock comprising ethanol to dehydration to produce an olefins stream comprising ethylene and water;
b) drying at least part of the olefins stream;
said process being **characterized by** the fact of adding a reducing agent to the at least part of the olefins stream prior to drying it.

2. Process according to preceding claim, wherein the ethanol comprises a biomass-derived ethanol.

3. Process according to any of the preceding claims, wherein the olefins stream is quenched in a quench device before being dried in step (b).

4. Process according to the preceding claim, wherein the quench uses at least one quench medium that is pH adjusted by adding an alkaline, preferably caustic.

5. Process according to the preceding claim, wherein the reducing agent is active in alkaline media and is at least partly added to the alkaline quench medium.

6. Process according to the preceding claim, wherein the reducing agent is a metal hydride or a reducing sulfur compound.

7. Process according to preceding claim, wherein the reducing agent is a metal hydride.

8. Process according to preceding claim, wherein the reducing agent is sodium borohydride.

9. Process according to claim 6, wherein the reducing agent is sodium bisulfite.

10. Process according to any of the preceding claims, wherein the olefins stream is compressed before being dried in step (b).

11. Process according to any of the preceding claims, wherein the olefins stream dried in step (b) is further purified by substantially removing all the C3+ therefrom.

12. Use of ethylene produced by a process according to any of the preceding claims for making polyethylene by polymerization or for making DCE by direct chlorination and/or by oxychlorination.

13. Use according to the preceding claim, comprising the steps of:
a) subjecting a feedstock comprising ethanol to dehydration to produce an olefins stream comprising ethylene and water;
b) adding a reducing agent to at least part of this olefins stream to obtain a reduced olefins stream;
c) drying said reduced olefins stream to obtain a dry reduced olefin stream;
d) feeding at least part of said dry reduced olefins stream to a direct chlorination unit for making DCE.

14. Use according to the preceding claim, wherein part of the olefins stream is not reduced and dried, and said part is fed to an oxychlorination unit for making DCE in an integrated unit making DCE both by direct chlorination and by oxychlorination.

15. Use according to claim 13 or 14, wherein part of the dry reduced olefins stream is fed to an oxychlorination unit for making DCE in an integrated unit making DCE both by direct chlorination and by oxychlorination.
